# EUROPEAN PATENT SPECIFICATION

(11) **EP 3 870 133 B1**
(45) Date of publication and mention of the grant of the patent: **18.06.2025**
(21) Application number: 19828347.5
(22) Date of filing: 26.10.2019
(51) Int. Cl.: A61K 6/58, A61L 27/46, A61L 27/56, A61L 27/58

(54) **BIOLOGICAL BARRIER MEMBRANE**
BIOLOGISCHE SPERRMEMBRAN
MEMBRANE BARRIÈRE BIOLOGIQUE

(30) Priority: 26.10.2018 PL 42755418
(43) Date of publication of application: 01.09.2021
(73) Proprietor: Instytut Wysokich Cisnien Polskiej Akademii Nauk, 01-142 Warszawa (PL)
(72) Inventor: HIGUCHI, Julia, 02-302 Warszawa (PL); FORTUNATO, Giuseppino, 9000 St.Gallen (CH); WOZNIAK, Bartosz, 19-100 Mo ki (PL); CHODARA, Agnieszka, 03-101 Warszawa (PL); LOJKOWSKI, Witold, 02-792 Warszawa (PL); SZALAJ, Urszula, 03-042 Warsaw (PL)
(74) Representative: AOMB Polska Sp. z.o.o.
(86) International application number: PCT/PL2019/050057
(87) International publication number: WO 2020/085927

(56) References cited:
- WO-A1-2016/178174
- KR-A- 20090 114 063
- LINHUI QIANG ET AL: "Electrospun Porous PDLLA Fiber Membrane Coated with nHA", APPLIED SCIENCES, vol. 8, no. 5, 21 May 2018 (2018-05-21), pages 831, XP055669125, DOI: 10.3390/app8050831

## Description

### Technical Field

The object of the invention is a biological barrier membrane used in medicine for tissue separation, particularly during and after dental surgery.

### Background Art

It is commonly known that periodontal damage is a frequent occurrence in dentistry and dental surgery, which requires tissue separation using a barrier membrane. This damage is caused by disease, age, injury, or genetic factors, and it leads to physical and aesthetic dysfunction of the masticatory apparatus. One of the causes of periodontal damage is inflammation that destroys tooth-supporting tissue. The tooth-supporting tissue wastes away and the periodontal ligament and its surrounding bone are destroyed. The use of a separation membrane is also indicated in cases of maxilla or mandible damage due to incorrect tooth extraction or the need for screwing a titanium implant into the patient's bone. In such case, after the introduction of an implant, the cavity is sprinkled with a natural or synthetic bone powder, secured with a separation membrane, and immobilized using special pins. In addition, a bone substitute in the form of granules or porous blocks can be introduced into the bone cavity. Separation membrane covering of bone loss inhibits the epithelial downgrowth in place of the bone tissue undergoing regeneration. After about six months the bone cavity is filled while the separation membrane should undergo gradual dissolution. The procedure of using a barrier membrane for tissue separation is called Guided Tissue Regeneration (GTR) or Guided Bone Regeneration (GBR). Separation membranes used for such purposes should be primarily characterized by controlled biodegradation, which enables tissue regeneration on both its sides which is conducive to tissue growth while providing nourishment by enabling the free flow of nutrients and oxygen through the structure of the membrane.

Both non-biodegradable and fully biodegradable membranes are used in therapeutic practice. Biodegradable separation membranes are made of, among other things, animal-derived collagen type I and III, of porcine and bovine origin. They are non-porous or naturally porous and available in many sizes and thicknesses, from 0.3 to 2 mm. Currently, such membranes are the most widespread in clinical use. They are characterized by a mild degradation profile and satisfactory tissue growth. They ensure stable base attachment, although occasionally it is necessary to use fixation stitches/pins to prevent the barrier material sliding over tissues. An inconvenient feature of such separation membranes is the need for animal tissue extraction and the accompanying long process of removing lipids, protein remnants, or potential zoonotic pathogens. In order to eliminate the need for using animal-derived tissue, separation membranes made of synthetic biodegradable polymers were developed. Crystalline polylactide (PLA), semi-crystalline poly-L-lactide (PLLA), amorphous poly-D,L-lactide (PDLLA), polyglycolide (PGA), polylactide-glycolide (PLGA) copolymer, and polycaprolactone (PCL) are typically used for this purpose, producing solid fibrous or mesh separation membranes. They ensure good base attachment and the porosity constitutes a relative barrier for cells and protection against bacterial penetration. Decomposition products of barrier membranes made of aforementioned biodegradable polymers are fully metabolized in the body. There is, however, an increased risk of inflammation during biodegradation due to a local drop in tissue pH. Publication US6031148 discloses a separation material made of PLGA, consisting of a solid layer of this material, coated on both sides with fibres with a diameter from 25 to 30 µm. Publication WO2015/172212 describes a barrier material made of a PGA/PLLA composite obtained using the method of evaporating solvent from the solution poured into a mould, which can produce solid plates with a thickness from 0.0001 to 10 mm.

Coating medical implants with hydroxyapatite nanoparticles to improve tissue affinity of such implants is also well-known and practised. Fibrous implants with spontaneously oriented fibres obtained from biodegradable polymers using the method of electrospinning are well-known and used in tissue engineering, including implants containing hydroxyapatite nanoparticles attached to their fibres. Information about tissue implants made of hydroxyapatite-coated polymer fibres was disclosed in the publication written by R. Tavakoli-Darestani et al., titled Poly (lactide-co-glycolide) nanofibers coated with collagen and nano-hydroxyapatite for bone tissue engineering [Novelty in Biomedicine, Vol. 1, No. 1, 2013, pp. 8-15(8)]. This publication discloses a process of producing an electrospun structure from a PLGA polymer and subsequently coated with collagen and nano-hydroxyapatite. The fibrous structure (fabric) is obtained in the process of electrospinning using a 15% PLGA solution in the mixture of DMF/tetrahydrofuran (THF), producing fibres with a diameter of 887 nm (± 57 nm). Coating this fabric with collagen and hydroxyapatite was performed, in turn, using the plasma method and by immersion and leaving the fabric overnight in a 1% water suspension of nano-hydroxyapatite (nHA). The membrane obtained in this manner was characterized by a lattice of PLGA fibres with large collagen and hydroxyapatite agglomerates with significant dispersion.

The publication written by L. Qiang et al., titled Electrospun Porous PDLLA Fiber Membrane Coated with nHA [Applied Sciences 2018, 8(5), p. 831] discloses the process of manufacturing a fibrous electrospun structure (fabric) made of a hydroxyapatite-modified PDLLA polymer with a size under 100 nm. This fabric was characterized by an average pre-coating fibre thickness of 1.2 µm. The modification step involved immersing the polymer fabric in ethyl alcohol containing nano-hydroxyapatite. The fabric's material shrinks under the influence of ethyl alcohol and the obtained structure has polymer PDLLA fibres with loosely embedded large hydroxyapatite agglomerates that fill the fibrous structure's pores. The hydroxyapatite content by weight ranged from 4.1 to 11.2%. A drop in mechanical strength was observed when attempting to increase the hydroxyapatite content, resulting from the material's shrinkage and exposure to ethyl alcohol. KR 2009 0114063 A discloses nanofibrous membranes comprising a polyester made by e.g. electrospinning for use as periodontal bone implants. The polyester can be polylactide-co-glycolide, polylactic acid, polyglycolic acid or a blend thereof. The fiber thickness is 10-900 nm. The nanofiber membrane is coated with a calcium phosphate material having a Ca/P ratio between 1.75 and 1.50, such as a hydroxyapatite.

### Disclosure of Invention

The purpose of the invention was to obtain a separation membrane with better properties than previously known.

This purpose is met by a membrane according to the invention in the form of a thin, porous, and elastic fibrous structure made of spontaneously distributed polymer fibres made of a biodegradable polymer solution using the method of electrospinning. Within this structure, its constituent polymer fibres have a thickness of 1 to 3 µm and are coated with hydroxyapatite nanoparticles with a size under 100 nm and a molar ratio of calcium to phosphorus (Ca/P) within the range of 1.56 to 1.66. The invention consists in the fact that the polymer fibre material is a mixture of poly-DL-lactide (PDLLA) and polylactide-glycolide (PLGA) while the hydroxyapatite content within the membrane ranges from 10 to 25% by weight. The hydroxyapatite coating of polymer fibres has the form of a matrix of these fibres with a thickness ranging from 10 to 300 nm, advantageously from 10 to 50 nm, and the average size of matrix hydroxyapatite particles does not exceed 50 nm. The membrane's porosity ranges from 50 to 95%, advantageously from 60 to 90%, and the size of membrane pores ranges from 5 to 30 µm, advantageously from 5 to 20 µm. Water absorption capacity ranges from 300 to 600%, advantageously from 450 to 600%. The hydroxyapatite coating rate of polymer fibres is at least 40%. The specific surface area of the membrane material ranges from 5 m²/g to 20 m²/g, advantageously from 15 to 20 m²/g.

In one of variants of the membrane according to the invention, the mass content of both polymers in the fibrous structure's fibre material is equal.

In next variant of the membrane according to the invention, the mass content of poly-DL-lactide (PDLLA) in the fibrous structure's fibre material is at least 5%.

In next variant of the membrane according to the invention, the mass content of polylactide-glycolide (PLGA) in the fibrous structure's fibre material is at least 5%. In next variant of the membrane according to the invention, the fibre matrix is made up of two layers of hydroxyapatite, made of hydroxyapatite grains of two different sizes.

In next variant of the membrane according to the invention, the external layer of the matrix is made up of hydroxyapatite grains with a size no larger than 20 nm.

In another variant of the membrane according to the invention, its thickness in the dry state ranges from 0.1 mm to 2 mm, advantageously from 0.3 to 1 mm.

A method according to the invention includes a first step, which produces a thin, porous, and elastic fibrous structure from spontaneously distributed polymer fibres with a thickness of 1 to 3 µm using the method of electrospinning from a biodegradable polymer solution, and a second step, where polymer fibres of the fibrous structure produced in the first step are coated with a layer of hydroxyapatite by immersing the produced fibrous structure in a suspension, which is made up of a liquid dispersing phase and the dispersed phase in the form of grains of synthetic hydroxyapatite with an average size no larger than 100 nm and a molar ratio of calcium to phosphorus (Ca/P) ranging from 1.56 to 1.66. The invention consists in the fact that the first step uses a solution made of a mixture of poly-DL-lactide (PDLLA) and polylactide-glycolide (PLGA) by dissolving one part by weight of the mixture of these polymers in five parts by weight of a volatile solvent in order to create the fibrous structure. This solution is used to produce a fibrous structure with a porosity ranging from 50 to 95%, advantageously from 60 to 90%, with pore size ranging from 5 to 30 µm, advantageously from 5 to 20 µm, and with thickness of its fibres ranges from 1 to 3 µm. The second step of this method produces a suspension whose dispersing phase is water and dispersed phase are hydroxyapatite particles with an average size no larger than 50 nm, and the mass content of the dispersed phase in the suspension is at most 0.5%, and the temperature of this suspension is maintained at the level of at most 30°C. After immersing the fibrous structure produced in the first step in the prepared suspension, an ultrasound wave with a power intensity ranging from 3 to 15 W/cm² and a duration of at least four minutes is generated near the polymer fibres of this structure.

In one of variants of the method according to the invention, the frequency of the ultrasound wave generated in the suspension is 20 kHz (± 2kHz).

In next variant of the method according to the invention, the polymer solvent is a mixture of nine parts chloroform (CHCl₃) and one part dimethylformamide (DMF). In next variant of the method according to the invention, the mass content of both polymers (PDLLA and PLGA) in the mixture of polymers, used in the first step to make the solution for producing fibres of the fibrous structure, is equal.

In next variant of the method according to the invention, the mass content of poly-DL-lactide (PDLLA) in the mixture of polymers, used in the first step to make the solution for producing fibres of the fibrous structure, is at least 5%.

In next variant of the method according to the invention, the mass content of polylactide-glycolide (PLGA) in the mixture of polymers, used in the first step to make the solution for producing fibres of the fibrous structure, is at least 5%.

In next variant of the method according to the invention, the second step produces the first and second suspension, in which the fibrous structure produced in the first step is immersed in turn, and an ultrasound wave is generated near the immersed polymer fibres, and the average size of hydroxyapatite particles of the first suspension is different from the average size of hydroxyapatite particles of the second suspension.

The structure and material of the membrane according to the invention is intended to block the penetration of cells of tissues being separated, while enabling their free growth on the surface thanks to the observed free flow of nutrients through it. This membrane exhibits a very high saline wettability (absorbency) exceeding 500% thanks to the obtained porosity and significant growth of the specific surface area compared to polymer fabrics without hydroxyapatite coating. The wetted membrane material has mechanical properties that are almost identical to the properties of natural gum tissue while being susceptible to bending, stitching, rolling, and cutting at a temperature of 37°C without losing hydroxyapatite coating on fibres. During the fibre degradation process, hydroxyapatite particles embedded on these polymer fibres neutralize the pH drop in surrounding tissue. Because the degradation time of the hydroxyapatite-coated polymer fibre is slower than observed in the case of the same fibres but without coating, and the drop in pH is slower as well, using the membrane according to the invention protects against tissue inflammation for a longer period.

Unexpectedly, it turned out that the method according to the invention preserves the desired fabric structure and does not destroy its very thin fibres while significantly and homogeneously coating them with hydroxyapatite and with a radical shortening of the finished membrane production process. Furthermore, the membrane biodegradation period within the body can be easily controlled by selecting the proportions of both polymers used to make the fibres, selecting the type of hydroxyapatite powder used to prepare the suspension, and selecting the parameters of the ultrasound wave.

### Brief Description of Drawings

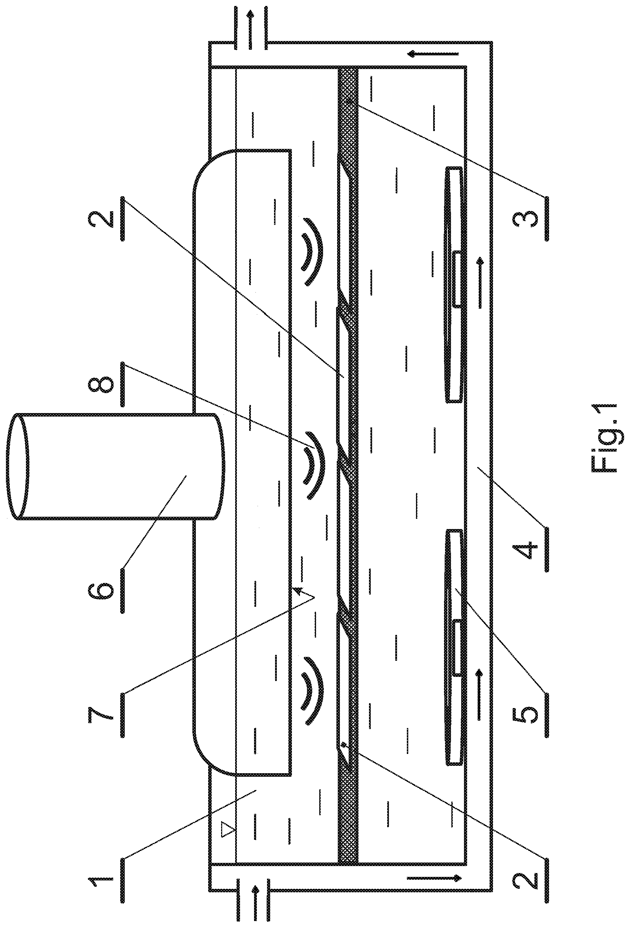

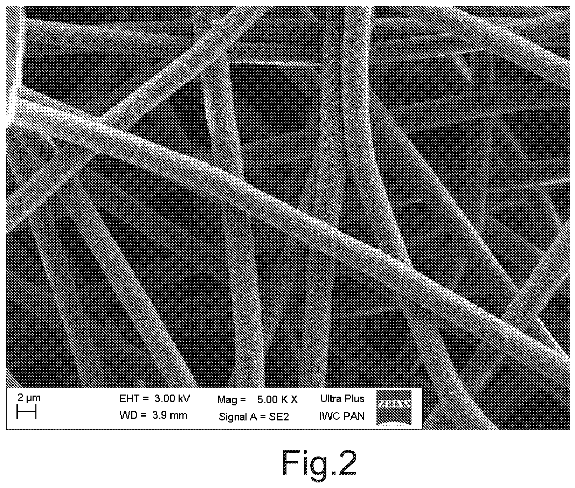

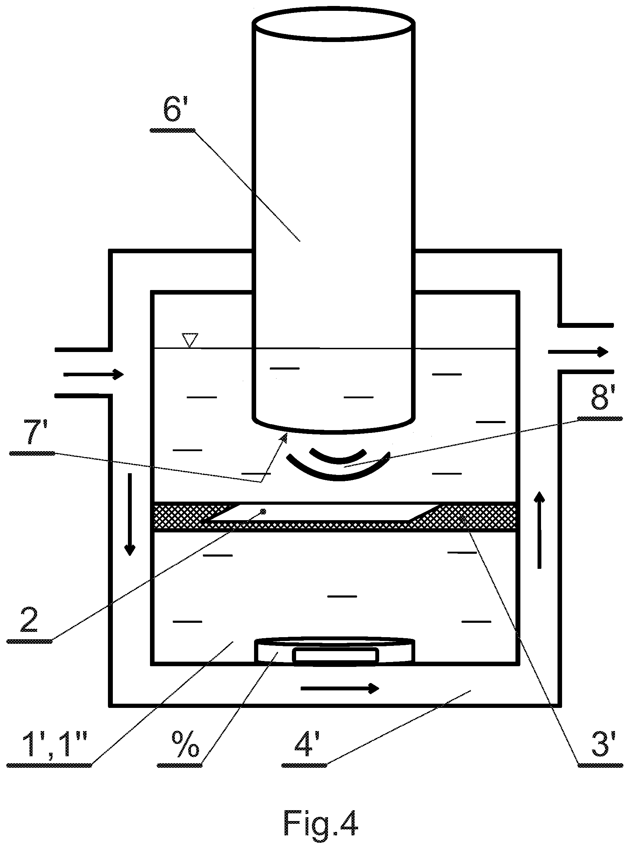

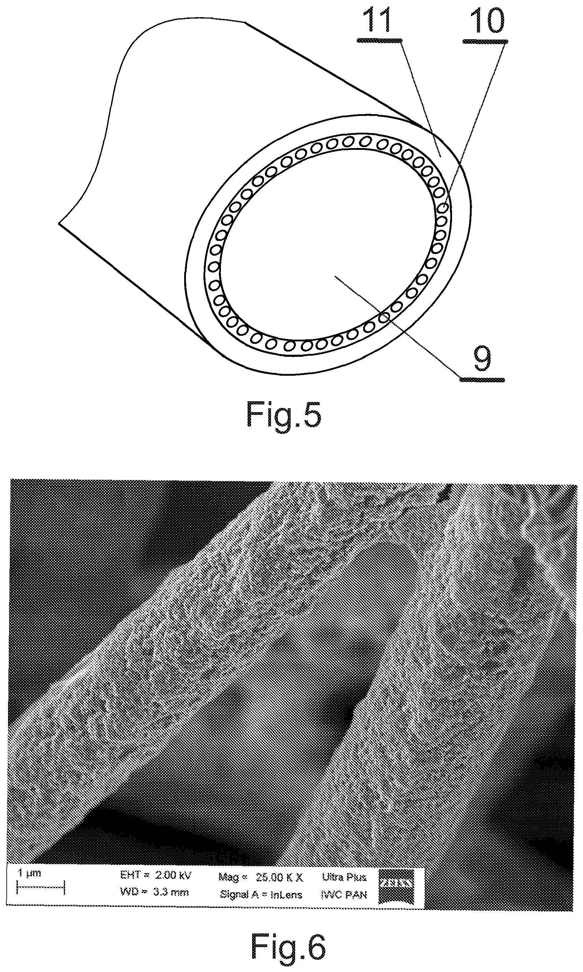

The invention is schematically presented in the drawing where Fig.1 shows a diagram of the setup for coating polymer fibres with hydroxyapatite, used in first, second, third and fourth examples. Fig. 2 shows a photo of hydroxyapatite-coated polymer fibres in the first example, obtained from a scanning electron microscope with a magnification of 5000x. Figs 3 show an assembly of photos from a scanning electron microscope of the structure from the first example and photos from an optical microscope during the measurement of the contact angle, wherein Fig.3A shows a pure fabric obtained during the first step of the membrane production process while Fig.3B shows a finished membrane. Fig.4 presents a diagram of the setup for coating polymer fibres with hydroxyapatite used in the second example. Fig.5 shows a diagram of the bilayer matrix of the polymer fibre from the second example, and Fig.6 shows a photo of this matrix obtained from a scanning electron microscope.

### Mode for Carrying out Invention

Four exemplary embodiments of the invention are described below. In each case, the process of producing four exemplary membranes according to the invention is made up of the fibrous structure production step and the following step of coating this structure with hydroxyapatite (HAp) particles.

In order to produce the four fibrous structures described below using the method of electrospinning, two biodegradable polymers belonging to the group of aliphatic polyesters were used, i.e. poly-DL-lactide (PDLLA), or polylactic acid, and polylactide-glycolide (PLGA), or lactic acid and glycolic acid copolymer. For the described purposes, a PLGA polymer was used with a lactide to glycolide ratio of 50:50 and a molecular mass of 30,000-40,000 Da, and a PDLLA polymer was used with a molecular mass of 300,000 Da, both manufactured by Polysciences from the United States. To obtain the solution used to produce polymer fibres, 2 g of the polymer mixture with the ratio described below was weighed and 9 ml of chloroform (CHCl₃) and 1 ml of dimethylformamide (DMF) were added, after which the mixture was mixed using a magnetic mixer in a closed vessel for about four hours. The obtained 15% solution of the polymer mixture was placed in a 10 ml disposable syringe which was placed in a syringe pump with the flow rate of 50 µl per minute. The entire system was placed in a laminar flow cabinet with a temperature ranging from 22 to 25°C and air humidity ranging from 20 to 40%. Then a metal needle with a diameter of 0.8 mm was connected to the syringe and a voltage of 12-14 kV was applied to the needle. The forming fibres were collected on a grounded rotating cylinder collector with a diameter of 25 mm located at a distance of 20 cm from the aforementioned metal needle and rotating with a velocity of 50 rpm until a fibrous structure (fabric) with a thickness of approx. 1 mm was produced on the collector. After completing the electrospinning process, the fabric in the form of a tube surrounding the collector was cut along its axis into 5 cm x 5 cm fragments, which were then washed twice with deionized water and left to dry completely for 24 hours in a laminar flow cabinet. The dried fragments of the fabric were weighed using an analytical scale with a precision of four decimal places.

During the step of coating the polymer fibres of the produced fabric, synthetic hydroxyapatite powder was used, i.e. a chemical compound with the formula Ca₁₀(PO₄)₆(OH)₂, where the calcium to phosphorus ratio (Ca/P) is greater than 1.55 and less than 1.67. In the following embodiments, two varieties of hydroxyapatite powder with the common trade name GoHAP were used, i.e. GoHAP type 3 (hereinafter GoHAP3) and GoHAP type 6 (hereinafter GoHAP6). The GoHAP3 powder was characterized by an average grain size of 19 nm ± 3 nm, a specific surface area of 120 m²/g ± 11 m²/g, and a skeletal density of 2.88 g/cm³ ± 0.03 g/cm³, while the GoHAP6 powder was characterized by an average grain size of 41 nm ± 6 nm, a specific surface area of 49 m²/g ± 5 m²/g, and a skeletal density of 3.00 g/cm³ ± 0.09 g/cm³.

The average size of these nanoparticles was determined by analysing the image obtained using a transmission electron microscope (TEM) using the dark-field method for at least 200 particles, and the average size was the diameter of the circle outlining the grain shape. The specific surface area is understood here as a scalar parameter expressing the area of a substance per its amount, measured by analysing the BET adsorption isotherms (Brunauer-Emmett-Teller isotherm).

The following embodiments state the properties of obtained separation membranes defined below:
- membrane water absorption capacity is its capacity to absorb water expressed as a percentage ratio of the mass of water absorbed by the membrane to the mass of this membrane in the dry state;
- membrane porosity specifies the ratio of pores, i.e. void spaces in its structure, specified numerically as a ratio of the total volume of these void spaces (pores) to the entire volume of the membrane, expressed in percent; when specifying open porosity, significant from the point of view of water absorption, only the volume of open pores is accounted for;
- porous membrane pore size is determined by the longest measured diagonal of the polygon created by the lattice of fibres in a scanning microscopy image (2D) and the average size of these pores measured using Capillary Flow Porometry;
- the coating rate of membrane polymer fibres by hydroxyapatite particles is a percentage fraction of the external surface of these fibres taken by these particles; in the following embodiments, it is determined by a scanning electron microscope (SEM) analysis as a measurement from twenty sample areas at a magnification of 5000x.

### Example 1

During the first step, in order to prepare the polymer solution for electrospinning, 1 g of the PDLLA polymer and 1 g of PLGA polymer were weighed and covered with the aforementioned solvent; the produced fibres were collected on a collector in the form of a tube of a diameter of 2.5 cm and rotated with a velocity of 50 rpm.

During the second step, the suspension 1 was prepared in which the dispersing phase was water and the dispersed phase was the GoHAP3 powder in the amount of 0.1% by weight. The dried fragments of the fabric 2 were immobilized on the stainless-steel mesh 3, and the mesh 3 with the fragment of fabric 2 was immersed in the prepared suspension 1 with a volume of 3,750 ml contained in the tank 4.The tank 4 also contained mixing parts 5 of a magnetic mixer not shown in the drawing in order to maintain a homogeneous suspension. Then the T-shaped sonotrode 6 connected to an ultrasound generator (not shown in the drawing) was placed in the suspension 1. The frontal area 7 of the sonotrode 6 with a length of 200 mm and a width of 5 cm was placed in front of fragments of the fabric 2, which were subjected for ten minutes to the ultrasound wave 8 with a frequency of 20 kHz (± 2kHz) and a power intensity of 3 W/cm² (± 2 W/cm²). The temperature of the suspension 1 was maintained at a level of 28-30°C using a flow cooling system within the walls of the tank 4. The temperature of the liquid in the tank 4 was constantly controlled using a thermocouple not shown in the drawing. After finishing the process, the hydroxyapatite-coated fragments of the fabric 2 were taken out of the suspension 1 and washed with deionized water, after which they were left to dry for 24 hours. The dried membranes were stored in a desiccator containing moisture-absorbing granules until the obtained material was characterized.

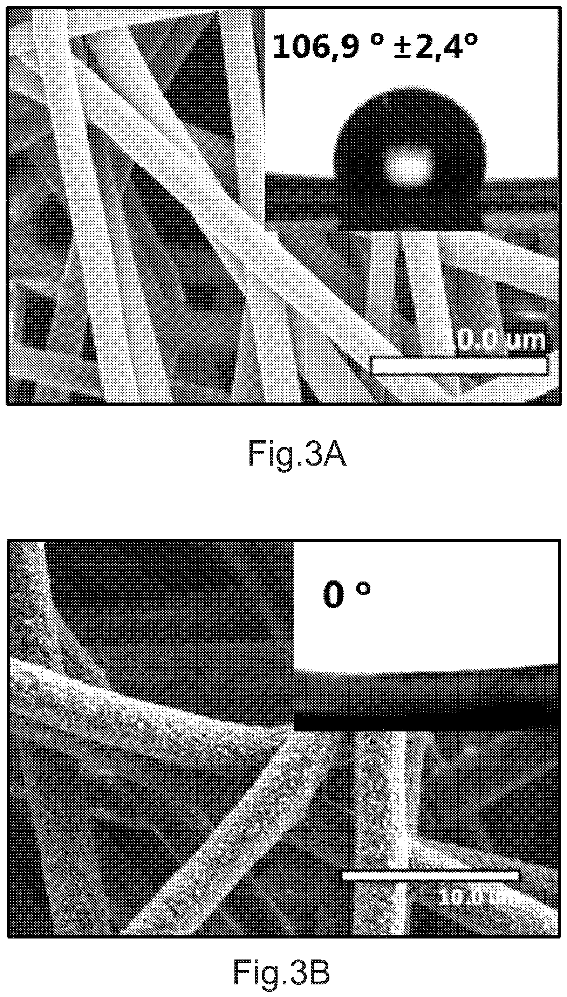

The membrane obtained in this embodiment was characterized by:
- hydroxyapatite content of 20% by weight;
- homogeneous hydroxyapatite layer;
- hydroxyapatite coating rate of 95%;
- water absorption capacity of 500% and contact angle of 0° (± 1°, Fig.3B);
- average porosity of 80-90%;
- average fibre thickness of 2 µm (± 0.5 µm);
- average hydroxyapatite layer thickness of 200 nm (± 50 nm);
- average pore size of 10 µm (± 5 µm);
- total degradation time in PBS and TRIS HCl media at a temperature of 37°C of five months;
- specific surface area of 15.26 m²/g (± 0.27 m²/g).

The membrane material was subjected to cellular cytotoxicity tests. In the cytotoxicity test, as per the PN-EN ISO 10993-5:2009 standard, NCTC clone 929 mouse fibroblast line cells in the MEM (Lonza) medium, and fetal bovine serum FBS (Euroclone) with an antibiotic solution (Lonza) were used. The measurement procedure involved tests on extracts after culturing in nutrients with serum at 37°C ± 1°C after 24 hours of exposure. Negative, positive, and reagent culture controls were used. According to the research expert report, the sample was deemed not toxic to cells.

### Example 2

During the first step, the fabric was produced from the PLGA and PDLLA polymer solution described in the first embodiment, collecting fibres on a collector in the form of a drum with a diameter of 20 cm and rotating with a velocity of 100 rpm. During the second step, the first water suspension 1' was produced, in which the dispersed phase was GoHAP6 in the amount of 0.1% by weight, and the second water suspension 1" was produced, containing 0.1% of the GoHAP3 powder by weight, i.e. analogous to the suspension 1 from the first embodiment. The dried fragment of the fabric 2' was immobilized on the stainless-steel mesh 3' and immersed in the stainless-steel tank 4'. The tank 4' was equipped with a liquid temperature stabilization system analogous to the one in the first embodiment, containing the mixing part 5 of the magnetic mixer and 450 ml of the first suspension 1' (GoHAP6). Then the head 7' of the cylinder sonotrode 6' with a diameter of 2 cm, connected to the ultrasound generator not shown in the drawing, was placed against the fragment of the fabric 2', after which this fragment of the fabric 2' was subjected for five minutes to the ultrasound wave 8' with a frequency of 20 kHz (± 2 kHz) and a power intensity of 12 W/cm² (± 2 W/cm²). Then the fragment of the fabric 2' already coated with the first type of hydroxyapatite was taken out of the suspension 1' and washed with deionized water, after which ultrasound coating of the membrane being produced was applied again in the second suspension 1" (GoHAP3) in the same conditions for another five minutes. After taking the finished membrane out of the second suspension 1", it was washed once with deionized water and left to dry for 24 hours. The obtained membrane was stored in a desiccator with moisture-absorbing granules until its properties were tested.

The separation membrane produced in this example had a bilayer matrix on polymer fibres 9, which was made up of an internal layer 10 of the GoHAP6 hydroxyapatite and an external layer 11 of the GoHAP3 hydroxyapatite. This membrane was characterized by the following properties:
- hydroxyapatite coating rate of 85%;
- hydroxyapatite content of 15% by weight;
- saline absorption and water absorption capacity of 550%;
- average open porosity of 80-90%;
- average thickness of its fibres was 2.5 µm (± 1 µm);
- average thickness of the hydroxyapatite layer was 150 nm (± 50 nm).

### Example 3

During the first step, a 15% solution of polymers was produced from their mixture containing 95% (i.e. 1.9 g) of the PDLLA polymer and 5% (i.e. 0.1 g) of the PLGA polymer. The production of this fabric solution using the method of electrospinning took place in the same conditions as in the second embodiment.

During the second step, the obtained fragments of the fabric 2 were coated using ultrasound in the same conditions (GoHAP3 suspension) as in the first embodiment, and after washing the finished membrane with deionized water, it was left to dry for 24hours. The membrane produced in this example was characterized by the following properties:
- hydroxyapatite fibre coating rate of 87%;
- hydroxyapatite content of 14% by weight;
- saline absorption and water absorption capacity of 500%;
- average open porosity of 80-90%;
- average fibre thickness of 2 µm (± 1 µm);
- average thickness of the hydroxyapatite layer was 150 nm (± 50 nm).

### Example 4

During the first step, a fabric was produced from a mixture containing 95% (i.e. 1.9 g) of the PLGA polymer and 5% (i.e. 0.1 g) of the PDLLA polymer. The production of this fabric solution using the method of electrospinning took place in the same conditions as in embodiments two and three.

During the second step, the obtained fragments of the fabric 2 were coated using ultrasound in the same conditions as in embodiments one and three, and the source of hydroxyapatite was a 0.1% water suspension of the GoHAP6 powder, i.e. the first suspension 1" in the second embodiment. The finished membrane, washed with deionized water, dried out, and stored in conditions described in pre-vious examples, had the following properties:
- hydroxyapatite coating rate of 90%;
- hydroxyapatite content of 15% by weight;
- saline absorption and water absorption capacity of 500%;
- average open porosity of 80-90%;
- average fibre thickness of 2 µm (± 1 µm);
- average thickness of the hydroxyapatite layer on polymer fibres was 150 nm (± 50 nm).

## Claims

1. A biological barrier membrane in the form of a thin, porous, and elastic fibrous structure made of spontaneously distributed polymer fibres fabricated from a biodegradable polymer solution using the method of electrospinning, in which the constituent polymer fibres are coated with hydroxyapatite nanoparticles with a size under 100 nm and a molar ratio of calcium to phosphorus (Ca/P) within the range of 1.56 to 1.66, **characterized in that:**
- the thickness of the polimer fibres ranges from 1 to 3 µm;
- the material of the polymer fibres (9) is a mixture of poly-DL-lactide (PDLLA) and polylactide-glycolide (PLGA);
- the hydroxyapatite content in the membrane is 10 to 25% by weight;
- the coating of polymer fibres (9) with hydroxyapatite has the form of a homogeneous coating on these fibres (9) whose thickness ranges from 10 to 300 nm, advantageously from 10 to 50 nm;
- the average size of hydroxyapatite particles is 50 nm at most;
- the membrane's porosity ranges from 50 to 95%, advantageously from 60 to 90%, and the size of membrane pores ranges from 5 to 30 µm, advantageously from 5 to 20 µm;
- water absorption capacity ranges from 300 to 600%, advantageously from 450 to 600%;
- the hydroxyapatite coating rate of polymer fibres is at least 40%;
- the specific surface area of the membrane material ranges from 5 m²/g to 20 m² g, advantageously from 15 to 20 m²/g.

2. The membrane according to claim 1, **characterized in that** the mass content of both polymers in the fibrous structure's fibre material is equal.

3. The membrane according to claim 1, **characterized in that** the mass content of poly-DL-lactide (PDLLA) in the fibrous structure's fibre material is at least 5%.

4. The membrane according to claim 1, **characterized in that** the mass content of polylactide-glycolide (PLGA) in the fibrous structure's fibre material is at least 5%.

5. The membrane according to one of claims from 1 to 4, **characterized in that** the fibre (9) matrix is made up of two layers (10, 11) of hydroxyapatite, made of hydroxyapatite grains of two different sizes.

6. The membrane according to claim 5, **characterized in that** the external layer (11) of the matrix is made up of hydroxyapatite grains with a size no larger than 20 nm.

7. The membrane according to one of claims from 1 to 6, **characterized in that** its thickness in the dry state ranges from 0.1 mm to 2 mm, preferably from 0.3 to 1 mm.

8. A method of producing a biological barrier membrane, including a first step, which produces a thin, porous, and elastic fibrous structure from spontaneously distributed polymer fibres using the method of electrospinning from a biodegradable polymer solution, and a second step, where polymer fibres of the fibrous structure produced in the first step are coated with a layer of hydroxyapatite by immersing the produced fibrous structure in a suspension, which is made up of a liquid dispersing phase and the dispersed phase in the form of grains of synthetic hydroxyapatite with an average size no larger than 100 nm and a molar ratio of calcium to phosphorus (Ca/P) ranging from 1.56 to 1.66, **characterized in that** the first step uses a solution made of a mixture of poly-DL-lactide (PDLLA) and polylactide-glycolide (PLGA) by dissolving one part by weight of the mixture of these polymers in five parts by weight of a volatile solvent in order to create the fibrous structure whose porosity ranges from 50 to 95%, advantageously from 60 to 90%, the size of its pores ranges from 5 to 30 µm, advantageously from 5 to 20 µm, and the thickness of its fibres ranges from 1 to 3 µm, while the second step produces a suspension whose dispersing phase is water and dispersed phase are hydroxyapatite particles with an average size no larger than 50 nm, and the mass content of the dispersed phase in the suspension is at most 0.5%, and the temperature of this suspension is maintained at the level of at most 30°C, whereas after immersing the fibrous structure produced in the first step in the prepared suspension, an ultrasound wave with a power intensity ranging from 3 to 15 W/cm² and a duration of at least four minutes is generated near the polymer fibres of this structure.

9. The method according to claim 8, **characterized in that** the frequency of the ultrasound wave generated in the suspension is 20 kHz (± 2kHz).

10. The method according to claim 8 or 9, **characterized in that** the polymer solvent is a mixture of nine parts chloroform (CHCl₃) and one part dimethylformamide (DMF).

11. The method according to one of claims from 8 to 10, **characterized in that** the mass content of both polymers (PDLLA and PLGA) in the mixture of polymers, used in the first step to make the solution for producing fibres of the fibrous structure, is equal.

12. The method according to one of claims from 8 to 10, **characterized in that** the mass content of poly-DL-lactide (PDLLA) in the mixture of polymers, used in the first step to make the solution for producing fibres of the fibrous structure, is at least 5%.

13. The method according to one of claims from 8 to 10, **characterized in that** the mass content of polylactide-glycolide (PLGA) in the mixture of polymers, used in the first step to make the solution for producing fibres of the fibrous structure, is at least 5%.

14. The method according to one of claims from 8 to 13, **characterized in that** the second step produces the first and second suspension, in which the fibrous structure produced in the first step is immersed in turn, and an ultrasound wave is generated near the immersed polymer fibres, and the average size of hydroxyapatite particles of the first suspension is different from the average size of hydroxyapatite particles of the second suspension.

## Patentansprüche

1. Eine biologische Barrieremembran in Form einer dünnen, porösen und elastischen Faserstruktur, die aus spontan verteilten Polymerfasern besteht, die aus einer biologisch abbaubaren Polymerlösung unter Verwendung des Verfahrens des Elektrospinnens hergestellt wurden, wobei die konstituierenden Polymerfasern mit Hydroxylapatit-Nanopartikel mit einer Größe unter 100 nm und einem Molverhältnis von Calcium zu Phosphor (Ca/P) im Bereich von 1,56 bis 1,66 beschichtet sind, **dadurch gekennzeichnet, dass:**
- die Dicke der Polymerfasern zwischen 1 und 3 µm liegt;
- das Material der Polymerfasern (9) eine Mischung aus Poly-DL-Lactid (PDLLA) und Polylactid-Glycolid (PLGA) ist;
- der Gehalt an Hydroxylapatit in der Membran 10 bis 25 Gew.-% beträgt;
- die Beschichtung der Polymerfasern (9) mit Hydroxylapatit die Form einer homogenen Beschichtung auf diesen Fasern (9) hat, deren Dicke von 10 bis 300 nm, vorzugsweise von 10 bis 50 nm, reicht;
- die durchschnittliche Größe der Hydroxylapatit-Partikel höchstens 50 nm beträgt;
- die Porosität der Membran von 50 bis 95 % reicht , vorzugsweise von 60 bis 90 %, und die Größe der Membranporen von 5 bis 30 µm reicht, vorzugsweise von 5 bis 20 µm;
- die Wasserabsorptionskapazität liegt im Bereich von 300 bis 600 %, vorzugsweise von 450 bis 600 %;
- der Anteil der Hydroxylapatit-Beschichtung der Polymerfasern beträgt mindestens 40 %;
- die spezifische Oberfläche des Membranmaterials liegt im Bereich von 5 m²/g bis 20 m² g, vorzugsweise von 15 bis 20 m²/g.

2. Die Membran nach Anspruch 1, **dadurch gekennzeichnet, dass** der Massengehalt der beiden Polymere im Fasermaterial der Faserstruktur gleich ist.

3. Die Membran nach Anspruch 1, **dadurch gekennzeichnet, dass** der Massengehalt an Poly-DL-Lactid (PDLLA) im Fasermaterial der Faserstruktur mindestens 5% beträgt.

4. Die Membran nach Anspruch 1, **dadurch gekennzeichnet, dass** der Massengehalt an Polylactid-Glycolid (PLGA) im Fasermaterial der Faserstruktur mindestens 5% beträgt.

5. Die Membran nach einem der Ansprüche 1 bis 4, **dadurch gekennzeichnet, dass** die Matrix der Fasern (9) aus zwei Schichten (10, 11) aus Hydroxylapatit besteht, die aus Hydroxylapatit-Körner zweier unterschiedlicher Größen hergestellt sind.

6. Die Membran nach Anspruch 5, **dadurch gekennzeichnet, dass** die äußere Schicht (11) der Matrix aus Hydroxylapatit-Körner mit einer Größe von höchstens 20 nm besteht.

7. Die Membran nach einem der Ansprüche 1 bis 6, **dadurch gekennzeichnet, dass** ihre Dicke im trockenen Zustand zwischen 0,1 mm und 2 mm, vorzugsweise zwischen 0,3 und 1 mm, liegt.

8. Ein Verfahren zur Herstellung einer biologischen Barrieremembran, umfassend einen ersten Schritt, der eine dünne, poröse und elastische Faserstruktur aus spontan verteilten Polymerfasern unter Verwendung des Verfahrens des Elektrospinnens aus einer biologisch abbaubaren Polymerlösung erzeugt, und einen zweiten Schritt, wobei die Polymerfasern der im ersten Schritt hergestellten Faserstruktur mit einer Schicht aus Hydroxylapatit beschichtet werden, indem die hergestellte Faserstruktur in eine Suspension getaucht wird, die aus einer flüssigen Dispersionsphase und der dispergierten Phase in Form von Körner aus synthetischem Hydroxylapatit mit einer durchschnittlichen Größe von nicht mehr als 100 nm und einem Molverhältnis von Calcium zu Phosphor (Ca/P) im Bereich von 1,56 bis 1,66 besteht, **dadurch gekennzeichnet, dass** in dem ersten Schritt eine Lösung verwendet wird, die aus einem Gemisch von Poly-DL-Lactid (PDLLA) und Polylactid-Glycolid (PLGA) hergestellt wird, indem ein Gewichtsteil des Gemischs dieser Polymere in fünf Gewichtsteilen eines flüchtigen Lösungsmittels aufgelöst wird, um die Faserstruktur zu erzeugen, deren Porosität im Bereich von 50 bis 95 %, vorzugsweise von 60 bis 90 %, die Größe der Poren zwischen 5 und 30 µm, vorzugsweise zwischen 5 und 20 µm, und die Dicke der Fasern zwischen 1 und 3 µm liegt, während im zweiten Schritt eine Suspension hergestellt wird, deren dispergierende Phase Wasser und die dispergierte Phase Hydroxylapatit-Partikel mit einer durchschnittlichen Größe von höchstens 50 nm sind, und der Massengehalt der dispergierten Phase in der Suspension höchstens 0,5 % beträgt und die Temperatur dieser Suspension auf höchstens 30 °C gehalten wird, während nach dem Eintauchen der im ersten Schritt hergestellten Faserstruktur in die vorbereitete Suspension eine Ultraschallwelle mit einer Leistungsintensität von 3 bis 15 W/cm² und einer Dauer von mindestens vier Minuten in der Nähe der Polymerfasern dieser Struktur erzeugt wird.

9. Das Verfahren nach Anspruch 8, **dadurch gekennzeichnet, dass** die Frequenz der in der Suspension erzeugten Ultraschallwelle 20 kHz (± 2kHz) beträgt.

10. Das Verfahren nach Anspruch 8 oder 9, **dadurch gekennzeichnet, dass** das Polymerlösungsmittel eine Mischung aus neun Teilen Chloroform (CHCl₃) und einem Teil Dimethylformamid (DMF) ist.

11. Das Verfahren nach einem der Ansprüche 8 bis 10, **dadurch gekennzeichnet, dass** der Massengehalt der beiden Polymere (PDLLA und PLGA) in der Polymermischung, die im ersten Schritt zur Herstellung der Lösung für die Herstellung der Fasern der Faserstruktur verwendet wird, gleich ist.

12. Das Verfahren nach einem der Ansprüche 8 bis 10, **dadurch gekennzeichnet, dass** der Massengehalt des Poly-DL-Lactid (PDLLA) in der Polymermischung, die im ersten Schritt zur Herstellung der Lösung für die Herstellung der Fasern der Faserstruktur verwendet wird, mindestens 5 % beträgt.

13. Das Verfahren nach einem der Ansprüche 8 bis 10, **dadurch gekennzeichnet, dass** der Massengehalt des Polylactid-Glycolid (PLGA) in der Polymermischung, die im ersten Schritt zur Herstellung der Lösung für die Herstellung der Fasern der Faserstruktur verwendet wird, mindestens 5 % beträgt.

14. Das Verfahren nach einem der Ansprüche 8 bis 13, **dadurch gekennzeichnet, dass** im zweiten Schritt die erste und die zweite Suspension hergestellt werden, in die die im ersten Schritt hergestellte Faserstruktur abwechselnd eingetaucht wird, und dass in der Nähe der eingetauchten Polymerfasern eine Ultraschallwelle erzeugt wird, und dass die durchschnittliche Größe der Hydroxylapatit-Partikel der ersten Suspension von der durchschnittlichen Größe der Hydroxylapatit-Partikel der zweiten Suspension verschieden ist.

## Revendications

1. Une membrane de barrière biologique sous la forme d'une structure fibreuse mince, poreuse et élastique composée de fibres de polimère réparties spontanément, fabriquées à partir d'une solution de polymère biodégradable à l'aide de la méthode d'électrofilage, dans laquelle les fibres de polimère constitutives sont revêtues de nanoparticules d'hydroxyapatite d'une taille inférieure à 100 nm et d'un rapport molaire entre le calcium et le phosphore (Ca/P) compris entre 1,56 et 1,66,:
- l'épaisseur des fibres de polimère varie de 1 à 3 µm ;
- le matériau des fibres de polymère (9) est un mélange de poly-DL-lactide (PDLLA) et de polylactide-glycolide (PLGA) ;
- la teneur en hydroxyapatite dans la membrane est de 10 à 25 % en poids ;
- le revêtement des **caractérisée en ce que** fibres de polymère (9) par l'hydroxyapatite se présente sous la forme d'un revêtement homogène sur ces fibres (9) dont l'épaisseur est comprise entre 10 et 300 nm, de préférence entre 10 et 50 nm ;
- la taille moyenne des particules d'hydroxyapatite est de 50 nm au maximum ;
- la porosité de la membrane est comprise entre 50 et 95 %, de préférence entre 60 et 90 %, et la taille des pores de la membrane est comprise entre 5 et 30 µm, de préférence entre 5 et 20 µm ;
- La capacité d'absorption d'eau est comprise entre 300 et 600 %, de préférence entre 450 et 600 % ;
- le taux de revêtement en hydroxyapatite des fibres de polymère est d'au moins 40 % ;
- la surface spécifique du matériau membranaire est comprise entre 5 m²/g et 20 m² g, de préférence entre 15 et 20 m²/g.

2. La membrane selon la revendication 1, **caractérisée en ce que** la teneur en masse des deux polymères dans le matériau fibreux de la structure fibreuse est égale.

3. La membrane selon la revendication 1, **caractérisée en ce que** la teneur en masse de poly-DL-lactide (PDLLA) dans le matériau fibreux de la structure fibreuse est d'au moins 5 %.

4. La membrane selon la revendication 1, **caractérisée en ce que** la teneur en masse de polylactide-glycolide (PLGA) dans le matériau fibreux de la structure fibreuse est d'au moins 5 %.

5. La membrane selon l'une des revendications de 1 à 4, **caractérisée en ce que** la matrice de fibres (9) est constituée de deux couches (10, 11) d'hydroxyapatite, constituées de grains d'hydroxyapatite de deux tailles différentes.

6. La membrane selon la revendication 5, **caractérisée en ce que** la couche externe (11) de la matrice est constituée de grains d'hydroxyapatite dont la taille n'excède pas 20 nm.

7. La membrane selon l'une des revendications de 1 à 6, **caractérisée en ce que** son épaisseur à l'état sec est comprise entre 0,1 mm et 2 mm, de préférence entre 0,3 et 1 mm.

8. Un procédé de production d'une membrane de barrière biologique, comprenant une première étape, qui produit une structure fibreuse mince, poreuse et élastique à partir de fibres de polymère distribuées spontanément en utilisant la méthode d'électrofilage à partir d'une solution de polymère biodégradable, et une deuxième étape, et une deuxième étape, où les fibres de polymère de la structure fibreuse produite dans la première étape sont revêtues d'une couche d'hydroxyapatite en immergeant la structure fibreuse produite dans une suspension composée d'une phase liquide dispersante et de la phase dispersée sous forme de grains d'hydroxyapatite synthétique dont la taille moyenne ne dépasse pas 100 nm et dont le rapport molaire entre le calcium et le phosphore (Ca/P) est compris entre 1,56 et 1,66,**caractérisé en ce que** la première étape utilise une solution constituée d'un mélange de poly-DL-lactide (PDLLA) et de polylactide-glycolide (PLGA) en dissolvant une partie en poids du mélange de ces polymères dans cinq parties en poids d'un solvant volatil afin de créer la structure fibreuse dont la porosité est comprise entre 50 et 95 %, de préférence entre 60 et 90 %, la taille de ses pores est comprise entre 5 et 30 µm, de préférence entre 5 et 20 µm, et l'épaisseur de ses fibres est comprise entre 1 et 3 µm, alors que la deuxième étape produit une suspension dont la phase dispersante est de l'eau et la phase dispersée sont des particules d'hydroxyapatite dont la taille moyenne ne dépasse pas 50 nm, et la teneur en masse de la phase dispersée dans la suspension est d'au plus 0,5 %, et la température de cette suspension est maintenue à un niveau d'au plus 30° C, après immersion de la structure fibreuse produite lors de la première étape dans la suspension préparée, une onde ultrasonore d'une intensité de 3 à 15 W/cm² et d'une durée d'au moins quatre minutes est générée à proximité des fibres de polymère de cette structure.

9. Le procédé selon la revendication 8, **caractérisé en ce que** la fréquence de l'onde ultrasonore générée dans la suspension est de 20 kHz (± 2kHz).

10. Le procédé selon la revendication 8 ou 9, **caractérisé en ce que** le solvant de polymère est un mélange de neuf parties de chloroforme (CHCl3) et d'une partie de diméthylformamide (DMF).

11. Le procédé selon l'une des revendications 8 à 10, **caractérisé en ce que** la teneur en masse des deux polymères (PDLLA et PLGA) dans le mélange de polymères, utilisé dans la première étape pour préparer la solution pour la production de fibres de la structure fibreuse, est égale.

12. Le procédé selon l'une des revendications 8 à 10, **caractérisé en ce que** la teneur en masse de poly-DL-lactide (PDLLA) dans le mélange de polymères, utilisé dans la première étape pour préparer la solution pour la production de fibres de la structure fibreuse, est d'au moins 5 %.

13. Le procédé selon l'une des revendications 8 à 10, **caractérisé en ce que** la teneur en masse de polylactide-glycolide (PLGA) dans le mélange de polymères, utilisé dans la première étape pour préparer la solution pour la production de fibres de la structure fibreuse, est d'au moins 5 %.

14. Le procédé selon l'une des revendications 8 à 13, **caractérisé en ce que** la deuxième étape produit la première et la deuxième suspension, dans lesquelles la structure fibreuse produite dans la première étape est immergée à son tour, et une onde ultrasonore est générée à proximité des fibres de polymère immergées, et la taille moyenne des particules d'hydroxyapatite de la première suspension est différente de la taille moyenne des particules d'hydroxyapatite de la deuxième suspension.
